# EUROPEAN PATENT APPLICATION

(11) **EP 3 378 340 A1**
(43) Date of publication of application: **26.09.2018**
(21) Application number: 18162330.7
(22) Date of filing: 16.03.2018
(51) Int. Cl.: A41B 13/06, A47G 9/08

(54) **BABY SLEEPING GARMENT**

(30) Priority: 24.03.2017 AU 2017901068
(71) Applicant: S & M Trading Pty Ltd, 3107 Lower Templestowe, Victoria (AU)
(72) Inventor: Villarreal, Lourdes, Lower Templestowe Victoria 3107 (AU)
(74) Representative: Vinsome, Rex Martin

(57) **Abstract**

Embodiments generally relate to a baby sleeping garment. The baby sleeping garment comprises a wrap portion configured to receive the body of the baby or infant and at least partially wrap around the body of the baby or infant; and at least one arm portion attached to the wrap portion around at least part of the arm pocket's perimeter to define at least one arm pocket sized to receive at least one arm of the baby or infant. The arm pocket has an opening configured to allow the at least one arm of the baby or infant to be inserted into the arm pocket.

## Description

### TECHNICAL FIELD

Described embodiments generally relate to garments for newborns and babies. In particular, described embodiments are directed to sleeping and swaddling garments for newborns and babies.

### BACKGROUND

While newborns and babies are in a REM state of sleep, they will often experience twitches and jerks, which are known as the startle reflex, or the moro reflex. This may cause the child to flail their arms sidewards and/or upwards before bringing their arms in to their chest in the foetal position. If the child is falling asleep when a startle reflex occurs, they may be caused to wake up.

In order to deal with this, newborns and babies are often swaddled or wrapped from birth until they grow out of the startle reflex. Babies often find comfort in being swaddled or wrapped up, as it gives them a feeling of security and echoes the feeling of being in the womb. An all-purpose blanket such as a receiving blanket may be used to swaddle a baby. Receiving blankets may be either square or rectangular in shape and are wrapped around the baby to help keep them feeling secure as well as to maintain their body heat. Newborns need to preserve their caloric intake for weight gain and can waste calories maintaining their own fragile temperatures.

It is desired to address or ameliorate one or more shortcomings or disadvantages associated with prior baby sleeping garments, or to at least provide a useful alternative thereto.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### SUMMARY

Some embodiments relate to a baby sleeping garment comprising:
a wrap portion configured to receive the body of the baby or infant and at least partially wrap around the body of the baby or infant; and
at least one arm portion attached to the wrap portion around at least part of the arm pocket's perimeter to define at least one arm pocket sized to receive at least one arm of the baby or infant;
wherein the arm pocket has an opening configured to allow the at least one arm of the baby or infant to be inserted into the arm pocket.

According to some embodiments, the arm pocket is configured to allow at least 90° of movement of the at least one arm of the baby or infant when the arm is in the arm pocket. In some embodiments the arm pocket is configured to allow at least 150° of movement of the at least one arm of the baby or infant when the arm is in the arm pocket. In some embodiments, the arm pocket opening is sized to inhibit the at least one arm of the baby or infant from inadvertently being released from the arm pocket.

According to some embodiments, the at least one arm pocket comprises two arm pockets, each arm pocket being configured to receive at least one arm of the baby or infant. According to some embodiments, the at least one arm pocket is configured to receive at least two arms of the baby or infant. In some embodiments, the at least one arm pocket is formed in an upside-down horseshoe shape.

According to some embodiments, the wrap portion extends beyond the perimeter of the at least one arm pocket in at least the right, left and downward directions, to define a right wrap portion, a left wrap portion and a bottom wrap portion. In some embodiments the top edge of the right wrap portion and the left wrap portion extend upwards and away from the at least one arm pocket, giving the top edge of wrap portion a generally "V" or "U" shape.

According to some embodiments the garment is configured to receive the body of a baby wearing a hip dysplasia harness.

In some embodiments the at least one arm pocket is positioned laterally around the centre of the wrap portion and adjacent to an upper edge of the wrap portion.

According to some embodiments, the at least one arm pocket is attached to the wrap portion by stitching or fusing. According to some embodiments, the at least one arm pocket and the wrap portion are produced as a unitary garment.

In some embodiments the width of the at least one arm pocket is between 30cm and 90cm. In some embodiments the width of the at least one arm pocket is between 50cm and 70cm. In some embodiments the width of the at least one arm pocket is around 60cm.

According to some embodiments, the dimensions of the wrap portion are between around 100cm x 100cm and between around 140cm x 140cm. In some embodiments the dimensions of the wrap portion are around 120cm x 120cm.

In some embodiments the baby garment comprises a lightweight, breathable and stretchy fabric. According to some embodiments, the baby garment comprises at least one of cotton jersey knit, bamboo jersey knit, thermal waffle, polyester, fleece, muslins, and flannel.

### BRIEF DESCRIPTION OF DRAWINGS

Embodiments are described in further detail below, by way of example and with reference to the accompanying drawings, in which:
Figures 1a to 1h show a number of infant sleeping positions ;
Figure 2 shows a flat layout of a sleeping garment according to some embodiments;
Figure 3 shows a flat layout of a first component of the sleeping garment of Figure 2;
Figure 4 shows a flat layout of a second component of the sleeping garment of Figure 2;
Figure 5 shows an infant placed in the sleeping garment of Figure 2;
Figure 6 shows a partially wrapped infant in the sleeping garment of Figure 2;
Figure 7 shows a partially wrapped infant in the sleeping garment of Figure 2;
Figure 8 shows a fully wrapped infant in the sleeping garment of Figure 2; and
Figure 9 shows a flat layout of a sleeping garment according to some alternative embodiments.

### DETAILED DESCRIPTION

Described embodiments generally relate to garments for newborns and babies. In particular, described embodiments are directed to sleeping garments for newborns and babies.

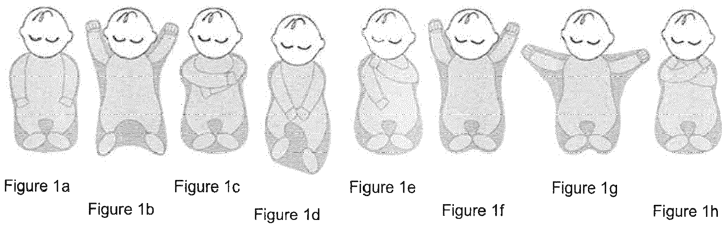

Figures 1a to 1h show a number of positions in which babies may sleep or rest. While some babies may sleep some of the time with their arms by their sides, as shown in Figure 1a, at times babies may stretch out their arms and legs as seen in Figures 1b, 1f and 1g. Babies may also cross their arms and "hug" themselves while they rest, as seen in figures 1c, 1d, 1e and 1h. When swaddling a baby using a receiving blanket or other traditional means, due to the movement of the babies arms during sleep the swaddling can become undone, or a baby's arms may escape the confines of the swaddling. Furthermore, many new parents find swaddling daunting and hard to get right, and may swaddle the baby too loosely so that the baby's arms can come out of the swaddling, or too tightly, restricting movement.

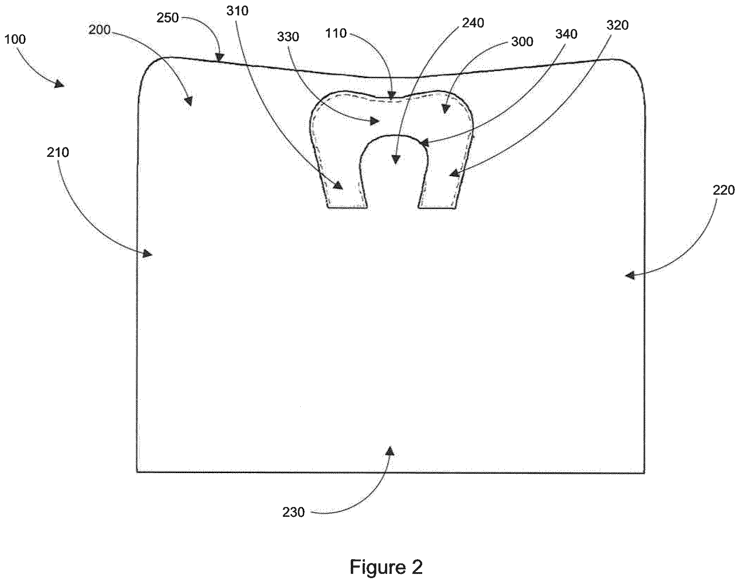

Figure 2 shows a sleeping garment 100 which can be used as a swaddle wrap blanket to swaddle babies from newborn to around 3 months old. Sleeping garment 100 allows swaddling of babies in the traditional way, but is designed to be easy to use and to keep babies arms always within the swaddle blanket, while allowing a range of arm motions. Sleeping garment 100 has a wrap portion 200 and an arm pocket 300, and is sized to allow swaddling of a baby. According to some embodiments, sleeping garment 100 may be around 120cm x 120cm. According to some embodiments, sleeping garment 100 may be around 100cm x 100cm, 140cm x 140cm, or another size that is suitable for swaddling a baby or infant.

By placing a baby in sleeping garment 100 with their arms inside arm pocket 300 and swaddling them with wrap portion 200, safety risks that are present due to incorrect swaddling may be minimised. For example, some parents may wrap their baby tightly in order to prevent the baby's arms from escaping the swaddle. However, this can lead to issues including hip dysplasia or issues with hip joint development, respiratory issues and stifling the startle reflex due to wrapping too tightly. Sleeping garment 100 confines the baby's arms to arm pocket 300, reducing the risk of arms escaping the swaddle, and allowing parents to wrap their baby less tightly.

When a baby's arms are positioned within arm pocket 300, the baby can position their arms in any of the ways illustrated in Figures 1a to 1h without their arms coming out of the wrap. According to some embodiments, each arm of a baby swaddled in sleeping garment 100 may have around 90° of movement. According to some embodiments, each arm of a baby swaddled in sleeping garment 100 may have more than 90° of movement, which may be more than 110°, more than 150° or around 180° in some embodiments. This is important for self-soothing, as some babies like to place their hands near their mouths, which they are able to do while swaddled in sleeping garment 100.

According to some embodiments, sleeping garment 100 may be made of a lightweight, breathable and stretchy fabric, such as cotton jersey knit, bamboo jersey knit, thermal waffle, polyester, fleece, muslins, flannel, and more eco-friendly options such as organic cotton or bamboo. The fabric may be selected to be comfortable for babies that suffer from eczema.

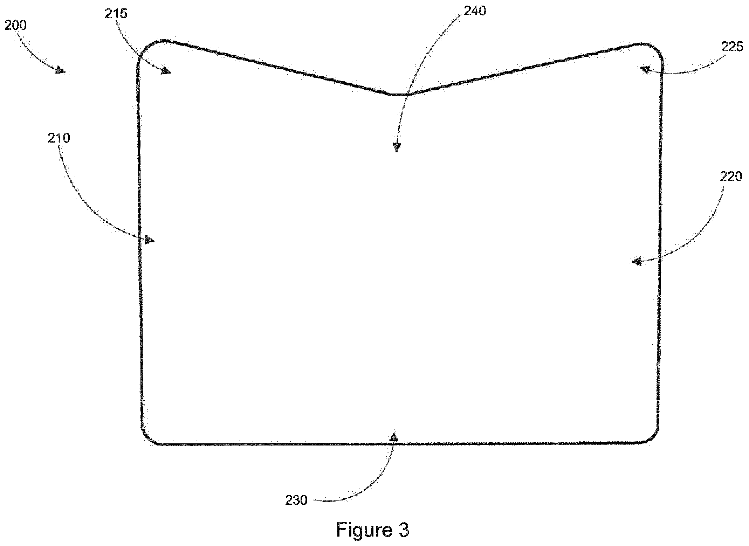

As shown in Figures 2 and 3, wrap portion 200 of sleeping garment 100 may be a square or rectangular shaped piece of cloth or material designed to at least partially wrap around the body a baby. Wrap portion 200 may have a left wrap segment 210 and a right wrap segment 220, as well as a bottom wrap segment 230. Wrap portion 200 may also have a baby placement segment 240, which may be proximate to arm pocket 300. According to some embodiments, baby placement segment 240 may be located around the centre of wrap portion 200, and may be at least partially surrounded by one or more arm pockets 300. Placing a baby on baby placement segment 240 may position the baby's arms in an area allowing them to be placed in arm pockets 300. According to some embodiments, wrap portion 200 may have a combination of one or more of a left wrap segment 210, a right wrap segment 220, and a bottom wrap segment 230. The wrap portions 210 220 and 230 may be of a length enabling each wrap portion to laterally encircle the body of a baby at least once.

According to some embodiments, wrap portion 200 may be shaped to have angled left and right wing segments 215 and 225, which may extend further upwards than baby placement segment 240, giving the top edge 250 of wrap portion 200 a "V" or "U" shape when the wrap portion is laid flat. Having the top edge 250 of wrap portion 200 angle upwards in this way allows for more room in the hip area of sleeping garment 100 when a baby is wrapped in the garment, which allows the garment to be worn by a baby that wears a hip dysplasia harness. This occurs due to the angle of the edges. As the angled edges are wrapped around a baby, more room is created around the hip area at the bottom of the wrap than if a wrap having a straight top edge is used.

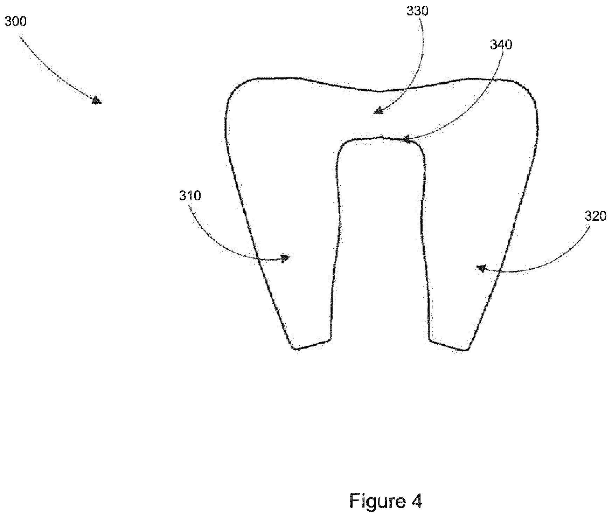

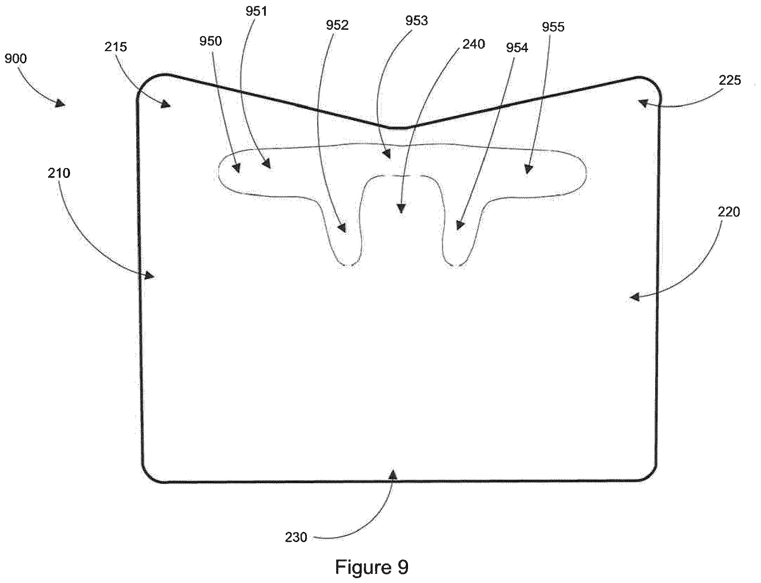

As illustrated in Figures 2 and 4, sleeping garment 100 further includes at least one arm pocket 300, sized and shaped to contain a baby's arms and hands while swaddled. Arm pocket 300 may be a shaped piece of material or cloth attached to lie substantially parallel to wrap portion 200 when flat, and may be attached by stitching 110, or may be fused or attached by another means. According to some embodiments, wrap portion 200 and arm pocket 300 may be produced as a unitary garment. While the Figures show one arm pocket 300 for holding two arms of a swaddled baby, according to some embodiments sleeping garment 100 may have two or more separate arm pockets 300 each designed to hold one arm. Each arm pocket may be substantially rectangular or oval in shape. Figure 9 shows an alternative arm pocket 900 having a different shape to arm pocket 300, which is described below in further detail.

When sleeping garment 100 comprises a single arm pocket 300, arm pocket 300 may be formed in a horseshoe shape, having two arm holding portions 310 and 320 extending downwards from an under-neck portion 330. Under-neck portion 330 may bridge the separate arm holding portions 310 and 320. Arm pocket 300 may be bonded to wrap portion 200 along the entire edge of arm pocket 300 aside from the internal edge 340 of the under-neck portion 330. This un-bonded edge allows access into arm pocket 300, so that a baby's arm can be placed between the layer of wrap portion 200 and the layer of arm pocket 300. Having the rest of the edge bonded to wrap portion 200 disallows the baby's hands to come out of sleeping garment 100, and prevents inadvertent scratching by the baby of its own face.

The width of arm pocket 300 across under-neck portion 330 may be between 20cm and 60cm, and may be between 30cm and 50cm in some embodiments. According to some embodiments, the width may be around 40cm. The length of arm pocket 300 from the top of under-neck portion 330 to the bottom of arm holding portion 310 or 320 may be between 20cm and 50cm, and may be between 30cm and 40cm in some embodiments. According to some embodiments, the length may be around 35cm.

Arm pocket 300 may be positioned around the lateral centre of and adjacent to an upper edge 250 of wrap portion 200. Arm pocket 300 may be positioned such that under-neck portion 330 is adjacent the upper edge 250, and that arm holding portions 310 and 320 extend from under-neck portion and away from the upper edge 250 of wrap portion 200. According to some embodiments, arm holding portions 310 and 320 may also extend slightly toward the upper edge 250 of wrap portion 200, and outwards toward the left and right edges of wrap portion 200, to allow a baby's arm positioned in arm holding portion 310 or 320 to move from down by its side to up around the level of its face without touching the edge of arm pocket 300. According to some embodiments, arm holding portions 310 and 320 may be of a width sized to allow a baby wrapped in sleeping garment 100 to move their arms through all of the positions illustrated in Figures 1a to 1h, including from next to its sides to above its head.

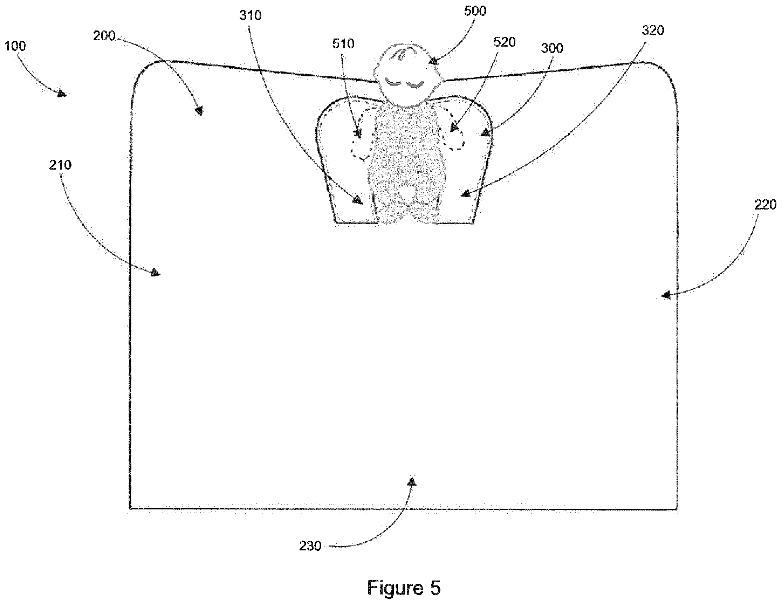

Figures 5 to 8 illustrate the method steps of wrapping a baby in sleeping garment 100. Figure 5 shows a baby 500 placed in baby placement segment 240, with left segment 210, right segment 220 and bottom segment 230 of wrap portion 200 extended flat. Arms 510 and 520 of baby 500 are placed in arm holding portions 310 and 320 of arm pocket 300, respectively. The baby's arms may be inserted into arm pocket 300 in a similar fashion as putting on jacket on the baby, except there are no openings for the hands to come through the ends of arm pocket 300.

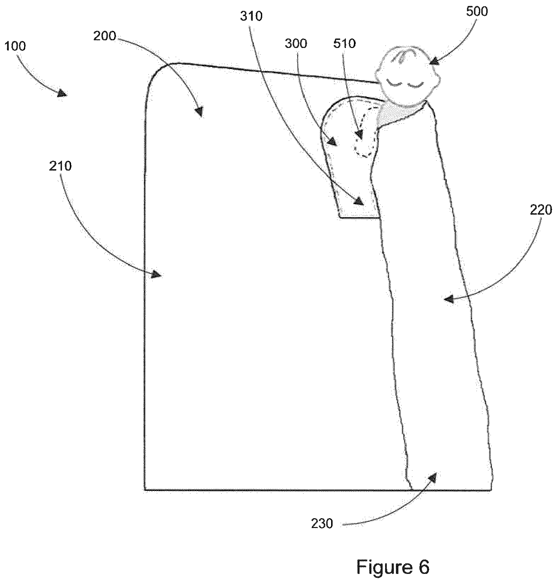

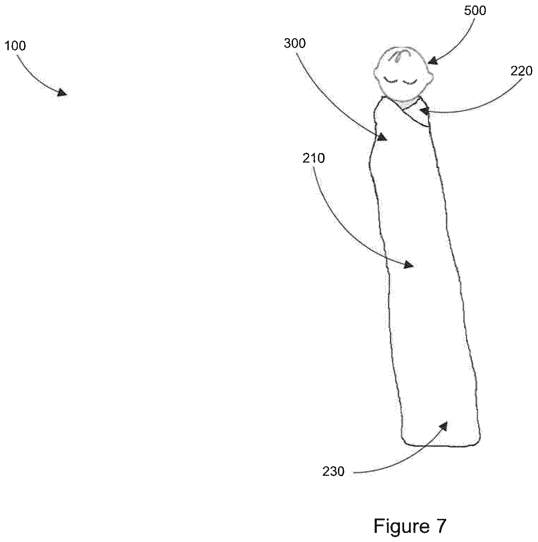

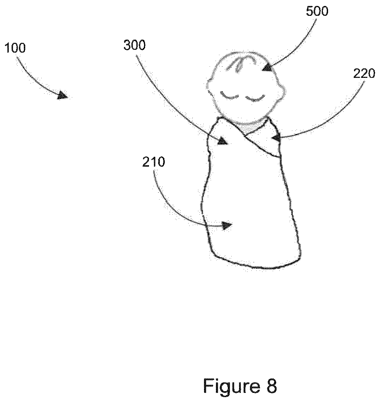

In Figure 6, right segment 220 of wrap portion 200 has been wrapped over and around the body of baby 500, with arm 520 remaining within arm holding portion 320. In Figure 7, left segment 210 of wrap portion 200 has been wrapped over and around the body of baby 500, with arm 510 remaining within arm holding portion 320. Finally, in Figure 8, bottom segment 230 has been wrapped under or over the body of baby 500, completing the swaddling process.

Figure 9 shows a sleeping garment 900 according to some alternative embodiments. Sleeping garment 900 may comprise a wrap portion 200 and an arm pocket 950. Wrap portion 200 may be a wrap portion as described above with reference to sleeping garment 100, and is sized to allow swaddling of a baby. According to some embodiments, sleeping garment 900 may be around 120cm x 120cm.

Arm pocket 950 may be positioned around the centre of and adjacent to an upper edge 250 of wrap portion 200. Arm pocket 950 may have downward arm holding portions 951 and 955, sideways arm holding portions 952 and 954, and an under-neck portion 953. Arm pocket 950 may be positioned such that under-neck portion 953 is adjacent upper edge 250, and that arm holding portions 951 and 955 extend from under-neck portion 953 and outwards towards the left and right edges of wrap portion 200. Arm holding portions 952 and 954 may extend from under-neck portion 953 downwards toward the bottom edge of wrap portion 20.

The width of arm pocket 950 from the outermost end of arm holding portion 951 to the outermost end of arm holding portion 955 may be between 30cm and 90cm, and may be between 50cm and 70cm in some embodiments. According to some embodiments, the width may be around 60cm. The length of arm pocket 950 from the top of under-neck portion 953 to the bottom of arm holding portion 952 or 954 may be between 10cm and 30cm, and may be between 15cm and 25cm in some embodiments. According to some embodiments, the length may be around 20cm.

Arm holding portions 951, 952, 954 and 955 may be of a width sized to contain a baby's arm. When positioning a baby in sleeping garment 900, the baby's arms may be positioned in arm pockets 951 and 955, so that the baby's arms are extended outwards as illustrated in Figure 1g, or the baby's arms may be positioned in arm pockets 952 and 954, so that the baby's arms are extended down by the baby's sides as illustrated in Figure 1a.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

## Claims

1. A baby sleeping garment comprising:
a wrap portion configured to receive the body of the baby or infant and at least partially wrap around the body of the baby or infant; and
at least one arm portion attached to the wrap portion around at least part of the arm pocket's perimeter to define at least one arm pocket sized to receive at least one arm of the baby or infant;
wherein the arm pocket has an opening configured to allow the at least one arm of the baby or infant to be inserted into the arm pocket.

2. The baby sleeping garment of claim 1, wherein the arm pocket is configured to allow at least 90° of movement of the at least one arm of the baby or infant when the arm is in the arm pocket.

3. The baby garment of claim 2, wherein the arm pocket is configured to allow at least 150° of movement of the at least one arm of the baby or infant when the arm is in the arm pocket.

4. The baby garment of any one of claims 1 to 3, wherein the arm pocket opening is sized to inhibit the at least one arm of the baby or infant from inadvertently being released from the arm pocket.

5. The baby garment of any one of claims 1 to 4, wherein the at least one arm pocket comprises two arm pockets, each arm pocket being configured to receive at least one arm of the baby or infant.

6. The baby garment of any one of claims 1 to 4, wherein the at least one arm pocket is configured to receive at least two arms of the baby or infant.

7. The baby garment of claim 6, wherein the at least one arm pocket is formed in an upside-down horseshoe shape.

8. The baby garment of any one of claims 1 to 7, wherein the wrap portion extends beyond the perimeter of the at least one arm pocket in at least the right, left and downward directions, to define a right wrap portion, a left wrap portion and a bottom wrap portion.

9. The baby garment of claim 8, where the top edge of the right wrap portion and the left wrap portion extend upwards and away from the at least one arm pocket, giving the top edge of wrap portion a generally "V" or "U" shape.

10. The baby garment of any one of claims 1 to 9, wherein the garment is configured to receive the body of a baby wearing a hip dysplasia harness.

11. The baby garment of any one of claims 1 to 10, wherein the at least one arm pocket is positioned laterally around the centre of the wrap portion and adjacent to an upper edge of the wrap portion.

12. The baby garment of any one of claims 1 to 11, including one of the following features: (i) wherein the at least one arm pocket is attached to the wrap portion by stitching or fusing; or (ii) wherein the at least one arm pocket and the wrap portion are produced as a unitary garment.

13. The baby garment of any one of claims 1 to 12, including one or more of the following features: (i) wherein the width of the at least one arm pocket is between 30cm and 90cm; or (ii) wherein the dimensions of the wrap portion are between around 100cm x 100cm and between around 140cm x 140cm; or (iii) wherein the baby garment comprises a lightweight, breathable and stretchy fabric; or (iv) wherein the baby garment comprises at least one of cotton jersey knit, bamboo jersey knit, thermal waffle, polyester, fleece, muslins, and flannel.

14. The baby garment of claim 13, including one or more of the following features: (i) wherein the width of the at least one arm pocket is between 50cm and 70cm; or (ii) wherein the dimensions of the wrap portion are around 120cm x 120cm..

15. The baby garment of claim 14, wherein the width of the at least one arm pocket is around 60cm.
